(19) Europäisches Patentamt
European Patent Office
Office européen des brevets

(11) EP 0 627 402 B1

(12) **FASCICULE DE BREVET EUROPEEN**

(45) Date de publication et mention de la délivrance du brevet:
**08.04.1998 Bulletin 1998/15**

(51) Int. Cl.$^6$: **C07C 59/52**, C07C 51/377

(21) Numéro de dépôt: **94401040.4**

(22) Date de dépôt: **10.05.1994**

(54) **Procédé d'obtention d'acides hydroxyphénylacétiques**

Verfahren zur Herstellung von Hydroxyphenylessigsäuren

Process for preparing hydroxyphenylacetic acids

(84) Etats contractants désignés:
**AT CH DE GB IE LI**

(30) Priorité: **28.05.1993 FR 9306434**

(43) Date de publication de la demande:
**07.12.1994 Bulletin 1994/49**

(73) Titulaire:
**SOCIETE FRANCAISE HOECHST**
**Société anonyme dite:**
**F-92800 Puteaux (FR)**

(72) Inventeurs:
- **Schouteeten, Alain**
  **F-95460 Ezanville (FR)**
- **Christidis, Yani**
  **F-75019 Paris (FR)**

(74) Mandataire: **Rinuy, Santarelli**
**14, avenue de la Grande Armée**
**75017 Paris (FR)**

(56) Documents cités:
**FR-A- 2 485 523** **US-A- 4 184 982**

- **PATENT ABSTRACTS OF JAPAN vol. 4, no. 144 (C-27) (626) 11 Octobre 1980 & JP-A-55 092 344 (NIPPON GOSEI KAGAKU KOGYO K.K.) 12 Juillet 1980**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 627 402 B1

Printed by Xerox (UK) Business Services
2.16.1/3.4

## Description

La présente demande concerne un procédé d'obtention d'acides hydroxyphénylacétiques.

Les acides o-, m- et p-hydroxyphénylacétiques, substitués ou non sur le noyau aromatique par un ou plusieurs substituants choisis parmi les halogènes ou les radicaux alcoyles ou alcoxyles sont des matières premières couramment utilisées en synthèse organique pour accéder à des produits présentant d'intéressantes propriétés physiologiques et/ou fongicides.

On connaît de très nombreux procédés pour obtenir ces acides hydroxyphénylacétiques. Parmi ceux-là, signalons l'hydrogènolyse soit par voie catalytique, soit par voie chimique, des acides hydroxymandéliques correspondants qui peuvent être issus de l'hydrolyse de l'hydroxymandélonitrile correspondant, ou pour certains d'entre eux, de la condensation de l'acide glyoxylique avec le phénol correspondant (cf Beil, 10, 410, 10, I, 199, 10-III, 1471 et 1474, 10-V, 1518, EP-A-536 960, FR-A-2 440 350, 2 427 322, 2 495 137, 2 638 740). Les procédés connus d'hydrogénolyse des acides hydroxymandéliques en acides hydroxyphénylacétiques correspondants présentent toutefois au niveau industriel de sérieux inconvénients en raison notamment du prix élevé de certains agents réactifs utilisés tels que l'acide iodhydrique, l'iode, le phosphore rouge, l'acide phosphoreux ou les sels d'étain ou de chrome (FR-A-2 426 669, 2 445 311, 2 588 869, GB-A-2 078 718, US-A-5 145 994, EP-A-028 375, 032 374, 526 672 et 224 401, JP-A-50/092 344, 58-052 242, Beil. 10, 187, 189, 190, 10,I, 81, 82, 10, II, 112, 10, III, 422, 428, 430, 10, IV, 536, 541, 543).

JP-A-55.92344 et FR-A-2 484 523 décrivent un procédé de préparation d'acide 4-hydroxyphénylacétique à une température comprise entre 50 et 120°C, à partir d'acide 4-hydroxymandélique de qualité élevée (cristallisé) car les impuretés gênent la réaction, en présence d'un catalyseur de type Palladium, Nickel ou Platine, en milieu acide carboxylique inférieur pouvant contenir de l'eau, en présence de 0,05 à 1 mole d'acide sulfurique par mole d'acide 4-hydroxymandélique, sous pression de plusieurs $kg/cm^2$.

Afin d'obvier à ces inconvénients, la demanderesse a découvert avec étonnement un nouveau procédé de préparation des acides hydroxyphénylacétiques.

La présente invention a pour objet un procédé de préparation d'un acide hydroxyphénylacétique de formule (I)

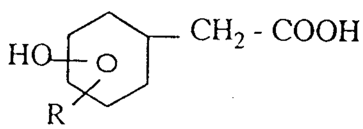

(I).

dans laquelle R représente un atome d'hydrogène ou un groupe alcoyle ou alcoxyle.

Le terme alcoyle peut désigner de préférence un alcoyle en $C_1$-$C_5$, par exemple un radical méthyle, éthyle, n-propyle, isopropyle, n-butyle, isobutyle, sec-butyle, tertiobutyle, pentyle.

le terme alcoxyle peut désigner de préférence un alcoxyle en $C_1$-$C_5$, par exemple un radical méthoxyle, éthoxyle, propoxyle, isopropoxyle, butoxyle, isobutoxyle, sec-butoxyle, tertiobutoxyle, pentaloxyle.

Selon l'invention, le procédé de préparation des acides hydroxyphénylacétiques de formule (I) et de leurs sels est caractérisé par le fait que l'on fait réagir en milieu aqueux, à une température supérieure ou égale à 50°C, l'acide hydroxymandélique correspondant, libre ou salifié de formule (II)

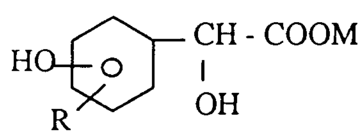

(II)

dans laquelle R a la signification donnée précédemment et M représente un atome d'hydrogène, de sodium ou de potassium en présence de nickel en poudre fine et d'un hydroxyde métallique de formule (III)

$$M_1OH \qquad (III)$$

où $M_1$ représente un atome de sodium ou de potassium et est identique à M dans le cas où M représente également un atome de sodium ou de potassium, de manière à ce que le milieu réactionnel présente un pH égal ou supérieur à 9, pour obtenir le sel correspondant de l'acide hydroxyphénylacétique de formule (I) que, si désiré, l'on déplace selon les méthodes usuelles pour obtenir l'acide correspondant que l'on isole ou, si désiré, l'on salifie selon les méthodes usuelles.

Dans des conditions préférentielles de mise en oeuvre de l'invention, le procédé ci-dessus décrit est réalisé de la manière suivante :

- à une température de 75 à 150°C,
- en présence de 1 à 2 atome-gramme de nickel en poudre fine par mole de produit de formule (II).

Du point de vue du taux de conversion et du rendement, on obtient les meilleurs résultats en utilisant un nickel activé commercialisé par la Société DEGUSSA sous la référence B 413/PEG. Pour sa conservation, ce nickel est enrobé avec du polyéthylèneglycol que l'on élimine juste avant son emploi par des lavages à l'eau chaude en atmosphère inerte.

En fin de réaction, le milieu réactionnel refroidi à la température ambiante est purgé avec de l'azote, puis le nickel est récupéré par filtration en atmosphère inerte et enfin l'acide hydroxyphénylacétique attendu est isolé du milieu réactionnel par des moyens connus en soi. Généralement, il est extrait du milieu réactionnel acidifié à pH=1 par de l'acide chlorhydrique concentré avec un solvant organique non miscible à l'eau tel que le dichlorométhane d'où il est récupéré par évaporation du solvant d'extraction. Si nécessaire, il est ensuite purifié soit par cristallisation, soit par distillation, soit enfin dans certains cas par l'intermédiaire de sa lactone.

Il est également possible de l'isoler par concentration du milieu réactionnel acidifié à pH=1 avec de l'acide chlorhydrique concentré, sous pression réduite, jusqu'à cristallisation de l'acide cherché que l'on isole ensuite par filtration.

Selon une variante de l'invention, le procédé décrit ci-dessus est réalisé en atmosphère d'hydrogène moléculaire, sous une pression de 1 à 5 bars, préférentiellement à une pression de 1,5 à 3 bars.

Dans des conditions encore plus préférentielles, le procédé ci-dessus décrit est mis en oeuvre de la manière suivante :

- à une température de 100 à 130°C,
- sous une pression de 1,5 à 3 bars d'hydrogène moléculaire,
- en présence de 1 à 1,75 atome-gramme de nickel par mole de produit de départ,
- en présence de 1 à 3 moles d'hydroxyde métallique $M_1OH$ par mole de produit de départ,
- en milieu aqueux à la concentration d'une mole de produit de départ par litre.

Parmi les acides hydroxyphénylacétiques de formule (I), on peut citer notamment :

- l'acide orthohydroxyphénylacétique,
- l'acide parahydroxyphénylacétique,
- l'acide hydroxy-4 méthoxy-3 phénylacétique.

Les produits de départ de formule (I) sont soit des produits aisément accessibles par des procédés connus tels que la condensation du cyanure de sodium sur l'hydroxybenzaldéhyde correspondant suivie de l'hydrolyse de l'hydroxymandélonitrile obtenu.

Les exemples suivants illustrent la présente invention sans toutefois la limiter.

**EXEMPLES 1 à 11**

Dans un hydrogénateur, on place une solution aqueuse contenant par kilogramme :

- 0,8 mole d'acide orthohydroxymandélique,
- 0,2 mole d'acide parahydroxymandélique,
- la quantité indiquée dans le tableau I d'hydroxyde de sodium.

Puis dans cette solution, on introduit en atmosphère inerte la quantité indiquée dans le tableau I de nickel activé (provenance DEGUSSA, référence B 413/PEG, enrobé avec du polyéthylèneglycol) préalablement lavé à l'eau chaude de manière à éliminer le polyéthylèneglycol. Après purge soigneuse de l'hydrogénateur avec de l'hydrogène, on chauffe l'hydrogénateur à la température indiquée pendant la durée indiquée à la pression ajustée à la valeur indiquée avec de l'hydrogène (les valeurs mentionnées dans le tableau I sont des pressions relatives à la pression atmosphérique). En fin de réaction, l'hydrogénateur est refroidi à la température ambiante, puis il est purgé avec de l'argon et le catalyseur est séparé par filtration en atmosphère inerte. Le filtrat est ensuite analysé par chromatographie pour déterminer le taux de transformation et le rendement en acide orthohydroxyphénylacétique. La sélectivité de la réaction est ensuite calculée selon l'équation 1 :

$$Sélectivité = \frac{Rendement(\%)}{Taux\ de\ transformation(\%)} \times 100 \qquad (équ.1)$$

**EXEMPLE 12**

Dans un hydrogénateur, on place une solution aqueuse contenant par kilogramme :

- 1 mole d'acide parahydroxymandélique pur,
- 2,5 moles d'hydroxyde de sodium,
- 100 g de nickel en poudre fine.

Après purge de l'hydrogénateur avec de l'hydrogène, on chauffe 4 heures à 125°C sous une pression de 3,4 bars. A ce stade, on dose dans le milieu réactionnel par chromatographie liquide à hautes performances (HPLC), 0,69 mole d'acide parahydroxyphénylacétique.

On refroidit le milieu réactionnel à la température ambiante puis on l'amène à la pression atmosphérique ; on filtre ensuite le nickel en atmosphère inerte et enfin on acidifie le filtrat à pH = 1 avec de l'acide chlorhydrique concentré. On le concentre ensuite sous pression réduite jusqu'au début de cristallisation de l'acide parahydroxyphénylacétique que l'on isole par filtration puis que l'on recristallise dans de l'eau. On obtient ainsi de l'acide parahydroxyphénylacétique pur.

**EXEMPLE 13**

On reproduit l'exemple 8 avec le nickel utilisé puis récupéré dans cet exemple. On obtient ainsi de l'acide orthohydroxyphénylacétique avec un rendement de 84,5% et un taux de transformation de 97% après 3 heures d'hydrogénation.

Un deuxième recyclage du nickel dans les mêmes conditions fournit un rendement de 91,5% en acide orthohydroxyphénylacétique avec un taux de transformation de 97%.

**EXEMPLE 14**

On chauffe à l'ébullition, sous agitation, pendant 24 heures, 150 g d'une solution aqueuse, amenée à pH = 11 avec de la lessive de soude et contenant 130 mmoles d'orthohydroxymandélate de sodium et 33 mmoles de parahydroxymandélate de sodium, en présence de 10,5 g de nickel en poudre fine.

En fin de réaction, on dose par HPLC, 71 mmoles d'acide orthohydroxyphénylacétique, 4 mmoles d'acide parahydroxyphénylacétique, et 31 mmoles d'acide orthohydroxymandélique dans le milieu réactionnel, soit un rendement de 55 % en acide orthohydroxyphénylacétique par rapport à la théorie calculée à partir de l'orthohydroxymandélate de sodium mis en oeuvre (taux de transformation 76 %, sélectivité 72 %).

TABLEAU I

| Température (°C) | 100 | | | | | 120 | | | | | 130 |
|---|---|---|---|---|---|---|---|---|---|---|---|
| Exemples | 1 | 2 | 3 | 4 | 5 | 6 | 7 | 8 | 9 | 10 | 11 |
| NaOH (moles/kg) | 1,75 | 2 | 2 | 3 | 4 | 2,5 | 2,5 | 2,5 | 3 | 3 | 2,5 |
| NI (atg/kg) | 1,7 | 1,7 | 1,7 | 1,7 | 1,7 | 0,23 | 0,57 | 1,7 | 1,1 | 1,7 | 1,1 |
| Pression (bar) | 1,5 | 25 | 1,5 | 1,5 | 1,5 | 2,8 | 2,8 | 2,8 | 2,8 | 2,8 | 3,5 |
| Durée (min) | 1230 | 240 | 1140 | 960 | 1380 | 460 | 580 | 225 | 270 | 210 | 140 |
| Taux de transformation (%) | 82,5 | 89 | 95 | 99 | 90 | 94 | 99 | 99 | 99,3 | 100 | 98,2 |
| Rendement (%) | 47 | 14 | 60 | 85 | 69 | 61 | 70 | 82 | 80 | 80 | 78,6 |
| Sélectivité (%) | 57 | 15,7 | 63 | 85,8 | 76,6 | 65 | 70,7 | 82,8 | 80,5 | 80 | 80 |

## Revendications

1. Procédé de préparation d'un acide hydroxyphénylacétique de formule (I)

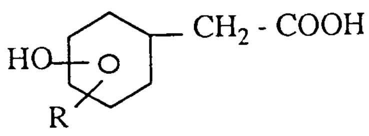

(I).

dans laquelle R représente un atome d'hydrogène ou un groupement alcoyle ou alcoxyle ainsi que de ses sels, caractérisé par le fait que l'on fait réagir, en milieu aqueux, à une température supérieure ou égale à 50°C, l'acide hydroxymandélique correspondant, libre ou salifié, de formule (II)

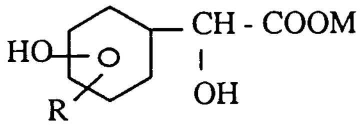

(II)

dans laquelle R a la signification donnée précédemment et M représente un atome d'hydrogène, de sodium, de potassium en présence de nickel en poudre fine et d'un hydroxyde métallique de formule (III)

$$M_1OH \quad (III)$$

où $M_1$ représente un atome de sodium ou de potassium et identique à M dans le cas où M représente également un atome de sodium ou de potassium, de manière à ce que le milieu réactionnel présente un pH égal ou supérieur à 9, pour obtenir le sel correspondant de l'acide hydroxyphénylacétique de formule (I) que, si désiré, l'on déplace selon les méthodes usuelles pour obtenir l'acide correspondant que l'on isole ou, si désiré, l'on salifie selon les méthodes usuelles.

**2.** Procédé selon la revendication 1, caractérisé par le fait qu'il est réalisé en atmosphère d'hydrogène moléculaire sous une pression de 1 à 5 bars.

**3.** Procédé selon l'une quelconque des revendications 1 et 2, caractérisé par le fait qu'il est effectué à une température comprise entre 75 et 150°C.

**4.** Procédé selon l'une quelconque des revendications 1 à 3, caractérisé par le fait qu'il est effectué en présence de 1 à 2 atome-gramme de nickel en poudre fine par mole de produit de formule (II).

**5.** Procédé selon l'une quelconque des revendications 1 à 4, caractérisé par le fait qu'il est effectué en présence de 1 à 3 moles d'hydroxyde métallique $M_1OH$ où $M_1$ représente un atome de sodium ou de potassium par mole de produit de formule (II).

**6.** Procédé selon l'une des revendications 1 à 5, caractérisé en ce que l'on prépare l'acide orthohydroxyphénylacétique.

## Claims

**1.** Preparation process for a hydroxyphenylacetic acid of formula (I):

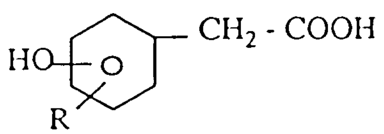

(I)

in which R represents a hydrogen atom or an alkyl or alkoxyl group as well as its salts, characterized in that the corresponding free or salified hydroxymandelic acid of formula (II):

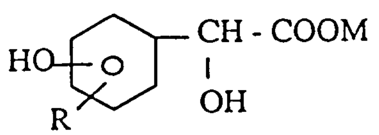

(II)

in which R has the meaning given previously and M represents a hydrogen, sodium, potassium atom is reacted in an aqueous medium, at a temperature greater than or equal to 50°C in the presence of finely powdered nickel and a metal hydroxide of formula (III)

$$M_1OH \quad (III)$$

where $M_1$ represents a sodium or potassium atom and is identical to M in the case where M also represents a sodium or potassium atom, in a such manner that the reaction medium has a pH equal to or greater than 9, in order to obtain the corresponding salt of the hydroxyphenylacetic acid of formula (I) which, if desired, is displaced according to the usual methods in order to obtain the corresponding acid which is isolated or, if desired, is salified according to the usual methods.

**2.** Process according to claim 1, characterized in that it is carried out in a molecular hydrogen atmosphere under a pressure of 1 to 5 bars.

**3.** Process according to any one of claims 1 and 2, characterized in that it is carried out at a temperature comprised

between 75 and 150°C.

4. Process according to any one of claims 1 to 3, characterized in that it is carried out in the presence of 1 to 2 atom-gram of finely powdered nickel per mole of product of formula (II).

5. Process according to any one of claims 1 to 4, characterized in that it is carried out in the presence of 1 to 3 moles of a metal hydroxide $M_1OH$ where $M_1$ represents a sodium or potassium atom per mole of product of formula (II).

6. Orthohydroxyphenylacetic acid prepared by the process according to any one of claims 1 to 5.

**Patentansprüche**

1. Verfahren zur Herstellung einer Hydroxyphenylessigsäure der Formel (I)

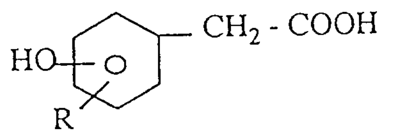

(I) ,

in welcher R ein Wasserstoffatom oder eine Alkyl- oder Alkoxylgruppe bedeutet, sowie deren Salze, dadurch gekennzeichnet, daß man in wäßrigem Medium bei einer Temperatur von mehr als oder gleich 50°C die entsprechende freie Hydroxymandelsäure oder deren Salz der Formel (II)

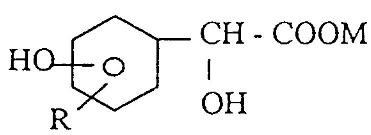

(II) ,

in welcher R die oben angegebene Bedeutung hat und M ein Wasserstoff-, Natrium-, Kaliumatom bedeutet, in Gegenwart von feinem Nickelpulver und einem Methallhydroxid der Formel (III)

$$M_1OH \qquad (III),$$

worin $M_1$ ein Natrium- oder Kaliumatom bedeutet und für den Fall mit M identisch ist, daß M ebenfalls ein Natrium- oder Kaliumatom bedeutet, in solcher Weise umsetzt, daß das Reaktionsmedium einen pH-Wert gleich oder größer als 9 hat, um das entsprechende Salz der Hydroxyphenylessigsäure der Formel (I) zu erhalten, das auf Wunsch nach üblichen Methoden umgelagert wird, um die entsprechende Säure zu erhalten, die isoliert oder auf Wunsch nach üblichen Methoden in ein Salz umgewandelt wird.

2. Verfahren nach Anspruch 1, dadurch gekennzeichnet, daß es in einer Atmosphäre von molekularem Wasserstoff bei einem Druck von 1 bis 5 bar durchgeführt wird.

3. Verfahren nach einem der Ansprüche 1 und 2, dadurch gekennzeichnet, daß es bei einer Temperatur zwischen 75 und 150°C durchgeführt wird.

4. Verfahren nach einem der Ansprüche 1 bis 3, dadurch gekennzeichnet, daß es in Gegenwart von 1 bis 2 Grammatom feinem Nickelpulver pro Mol des Produktes der Formel (II) durchgeführt wird.

5. Verfahren nach einem der Ansprüche 1 bis 4, dadurch gekennzeichnet, daß es in Gegenwart von 1 bis 3 Mol Metallhydroxid $M_1OH$, worin $M_1$ ein Natrium- oder Kaliumatom bedeutet, pro Mol des Produktes der Formel (II) durchgeführt wird.

6. Verfahren nach einem der Ansprüche 1 bis 5, dadurch gekennzeichnet, daß man Orthohydroxyphenylessigsäure herstellt.